# EUROPEAN PATENT SPECIFICATION

(11) **EP 1 733 756 B1**
(45) Date of publication and mention of the grant of the patent: **09.11.2011**
(21) Application number: 06076702.7
(22) Date of filing: 15.12.2004
(51) Int. Cl.: A61M 39/02

(54) **Flexible injection port**
Flexible Injektionsöffnung
Orifice d'injection souple

(30) Priority: 16.12.2003 US 737942
(43) Date of publication of application: 20.12.2006
(62) Divisional of application: 04257796.5
(73) Proprietor: ETHICON ENDO-SURGERY, INC., Cincinnati, Ohio 45242 (US)
(72) Inventor: Chen, How-Lun, Cincinnati Ohio 45242 (US); Conlon, Sean P., Loveland Ohio 45140 (US); Schulze, Dale R., Lebanon Ohio 45036 (US)
(74) Representative: Tunstall, Christopher Stephen

(56) References cited:
- EP-A- 0 203 633
- WO-A-00/27299
- US-A- 4 160 454
- US-A- 4 184 497
- US-A- 5 207 644

## Description

### Field of the Invention

This invention relates generally to the field of medicine, and more specifically to medical devices that are surgically implanted in a patient, and is particularly relevant to implantable injection or infusion ports such as used for chemotherapy and adjustable gastric band procedures.

### Background

Surgeons routinely implant subcutaneous injection ports in patients requiring long term, periodic fluid injections such as for chemotherapy and gastric band adjustments. The injection port connects to a flexible tube catheter to transport the fluid to the affected area (subclavian vein, etc.) or the gastric band. Current injection ports comprise a rigid metal or plastic housing, which is about 25mm in diameter and 15mm tall. A thick, silicone septum captured within the rigid housing covers an inner chamber that fluidly communicates with the catheter. The surgeon uses a hypodermic needle to inject fluid into the chamber through the silicone septum.

Typically the surgeon fastens the injection port with suture to fascia and beneath the fat and skin layers, primarily to prevent the port from flipping over, but also to prevent the injection port from migrating in the body. Since the septum is accessible from only one side of the injection port, flipping over requires interventional surgery to right the port for subsequent injections.

For some patients, the surgeon may place the injection port in the lower abdomen, thus burying the port beneath a fat layer that may be several centimeters thick. Usually a surgeon can locate the port with palpation alone. However, if there is a very thick, intervening fat layer, such as on extremely obese, gastric band patients, the surgeon must also use fluoroscopy, ultrasound, or other means to locate the port. Furthermore, the surgeon must inject the needle in a direction approximately perpendicular to the injection port, and hit the target area of the septum, which is only about 12-15mm in diameter. For some patients, the surgeon may place the injection port on the sternum or upper right chest, just beneath the skin layers. Although easy to locate with palpation, some patients regard the protruding port as uncomfortable or cosmetically objectionable.

US 4,184,497 discloses an injection port as defined in the preamble of claim 1.

What is needed, therefore, is a subcutaneously implantable injection port that is made of relatively soft and flexible materials, and ideally, that looks and feels more (than current injection ports) like a large, natural blood vessel. What is also needed is a subcutaneously implantable injection port that is penetrable with a hypodermic needle, independent of the orientation of the injection port in bodily tissue, and that is self-sealing when the needle is removed. What is further needed is a subcutaneously implantable injection port that a surgeon may position in the body more quickly and with less dissection than is required for conventional injection ports.

### Summary of the Invention

The present invention provides an injection port for subcutaneous placement within a body as recited in the claims.

### Brief Description of the Drawings

We present the specific, novel features of this invention in the appended claims. The reader may best understand, however, the organization and methods of operation of this invention by referring to the detailed description and the following drawings:
Figs. 1-7 and 10-12 do not represent the invention.
FIG. 1 is an isometric view of an injection port of the prior art;
FIG. 2 is a cross sectional view of the injection port of the prior art shown in FIG. 1;
FIG. 3 is an isometric view of a first example of a flexible injection port 30;
FIG. 4 is a sectional view of flexible injection port 30 shown in FIG. 3;
FIG. 5 is an enlarged, longitudinal sectional view of flexible injection port 30 penetrated by a hypodermic needle 100;
FIG. 6 is a cross sectional view of a second example of a flexible injection port 50;
FIG. 7 is a cross sectional view of a third example of a flexible injection port 60;
FIG. 8 is an isometric view of a fourth example of a flexible injection port 80, which is in accordance with the claimed invention;
FIG. 9 is a cross sectional view of flexible injection port 80;
FIG. 10 shows injection port 30 subcutaneously implanted near a fascia layer 124 in a patient;
FIG. 11 shows injection port 30 subcutaneously implanted near a skin layer 120 in a patient; and
FIG. 12 shows injection port 30 subcutaneously implanted in a fat layer 122 in a patient.

### Detailed Description of the Invention

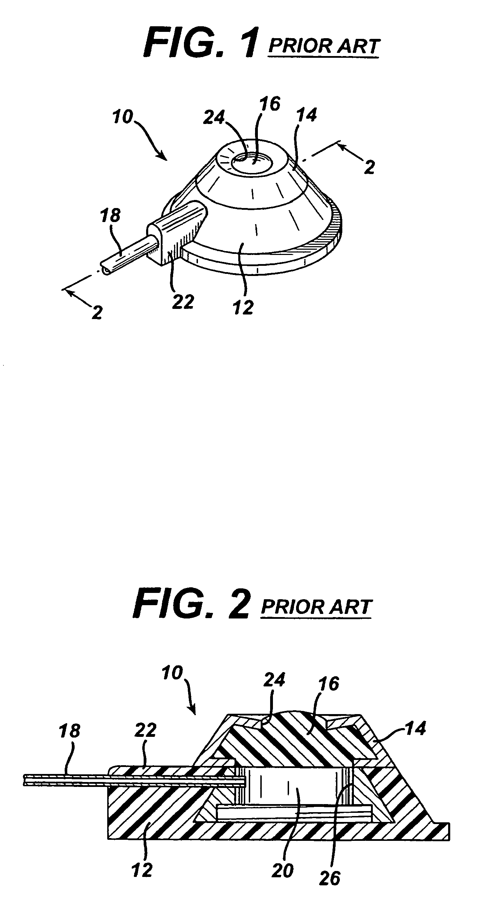

Referring now to the drawings, FIGS. 1 and 2 show an injection port 10 of the prior art. Injection port 10 generally has a truncated, conical configuration, and comprises a body portion 12, a housing 14, a seal element 16, and a catheter element 18. The body portion 12 is made of a flexible, rubberized material with a cavity 20 formed inside. A catheter support 22 integrally forms in body portion 12. Housing 14 is made of a corrosion resistant metal, and has a reduced, upwardly facing entry passage 24. Seal element 16 is made of a rubberized material, which is easily penetrable by a hypodermic needle or the like, and provides a penetrable seal for passage 24. Housing 14 and seal element 16 define an open cavity 20 in injection port 10 for receiving and containing a fluid. Catheter element 18 extends through catheter support 22 of body portion 12 and through housing 14 so that catheter element 18 extends into cavity 20 for providing communication between cavity 20 and the exterior of injection port 10 for dispensing fluid from the cavity 20 into the body of a patient.

A surgeon implants injection port 10 subcutaneously in a patient. To introduce a fluid such as a medication or a saline solution, the surgeon inserts a hypodermic needle or the like into the patient so that the tip of the needle passes through seal element 16 and into cavity 20. Due to the relatively small size of passage 24, each time the surgeon introduces a fluid into the patient, the surgeon must insert the needle through seal element 16 and the same localized area of the skin and tissue of the patient. Accordingly, seal element 16 may become significantly damaged and eventually develop a leak. Also, the localized skin area and underlying tissue may not heal in the desired manner. Further, because housing 14 is made of metal, it can cause barbing of the needle tip, causing increased trauma to the patient upon withdrawal of the needle. Still further, because of the truncated conical configuration of injection port 10 and the metallic construction of housing 14, injection port 10 can cause substantial discomfort to a patient, particularly if the area of the patient adjacent the injection port is accidentally bumped or bruised. In addition, because of the truncated conical configuration of injection port 10, it can cause a relatively unattractive mound on the body of a patient. Still further, since fluid can only be introduced in cavity 20 through passage 24, a surgeon must insert a needle into injection port 10 in substantially perpendicular relation to the skin so that often the adjacent area of tissue or skin of the patient cannot effectively support the needle.

When using injection port 10 of the prior art in a laparoscopic procedure such as implantation of a gastric band, it is necessary for the surgeon to assemble injection port 10 to catheter element 18 during the laparoscopic procedure. This is because injection port 10 is too large to pass through a standard size (12mm diameter) laparoscopic port, which is used for access to the stomach inside the abdominal cavity. The surgeon must introduce the gastric band and the catheter into the abdominal cavity without the injection port attached to the free end of the catheter. Once the surgeon has secured the gastric band around the stomach, the surgeon externalizes the free end of the catheter through the abdominal muscle and fascia layers, subcutaneous fat layer, and the skin to assemble the injection port to the free end of the catheter. Then the surgeon implants the injection port subcutaneously at the desired location on the patient's abdomen or chest. The surgeon must take extra time to assemble the injection port to the catheter. Also, the surgeon must skillfully connect the injection port to the catheter during less than ideal conditions. Consequently, there is the potential complication of an undiscovered leak developing at the connection of the catheter to the port.

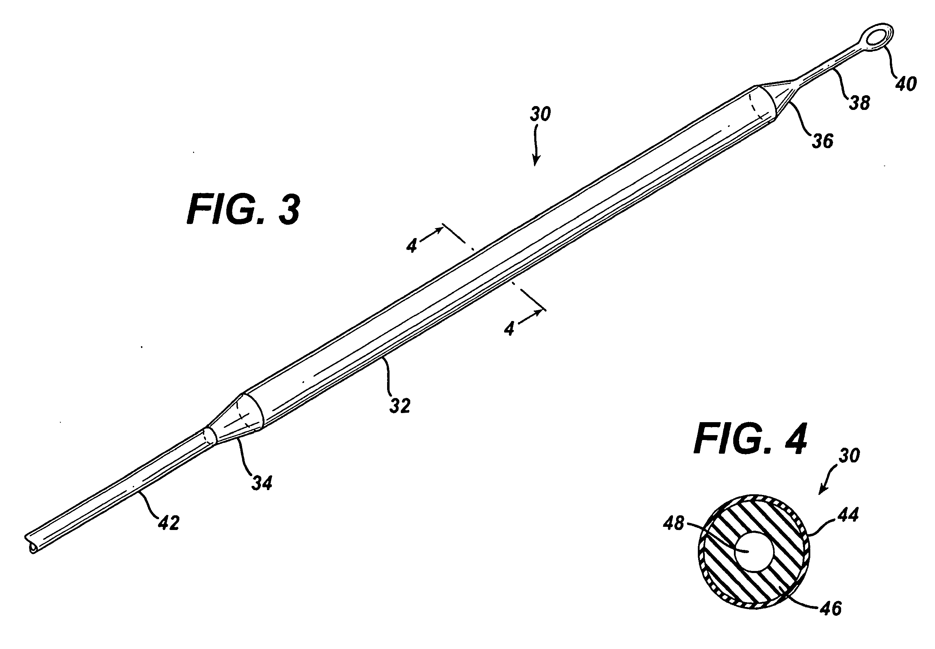

FIG. 3 is an isometric view of a first example of a flexible injection port or body 30, that generally comprises a first end 34, a second end 36, and a cylindrical injection portion 32 extending there between. A surgeon may use a hypodermic needle or the like to penetrate injection portion 32 and introduce a fluid such as a medication or saline solution into flexible injection port 30. Injection portion 32 self-seals when the surgeon removes the hypodermic needle. Injection portion 32 may have a length, but is not limited to, approximately 5 - 20 cm. Injection portion 32 may have a diameter, but is not limited to, approximately 5 - 12 mm. A catheter 42 attaches to first end 34 and distributes fluid injected into flexible injection port 30 to another portion of the patient's body. Catheter 42 is made from a silicone rubber or other biocompatible polymer such as known in the art for application to conventional injection ports, such as shown in FIGs 1 and 2. A tether 38 having an eye loop 40 extends from second end 36. A surgeon may use a conventional surgical grasping instrument to grasp tether 38, or a surgical suture tied to eye loop 40, or a combination of both grasper and suture, to facilitate placement of flexible injection port 30 in the body.

Although flexible injection port 30 is shown in FIG. 3 to be essentially straight, it is possible to construct it with a curved or non-straight shape in order to facilitate placement in the body, or to conform to the body anatomy at the implant location. Since flexible injection port 30 is made of relatively soft and flexible materials, the surgeon may temporarily straighten it, for example, when introducing it into the body through a laparoscopic port.

FIG. 4 is a cross sectional view of flexible injection port 30, taken at line 4-4 of injection portion 32 as shown in FIG. 3. At this location and anywhere along the length of injection portion 32, flexible injection port 30 includes an outer tube 44 that may exert a radial, compressive force on an inner tube 46. Flexible injection port 30 includes a fluid reservoir 48 that extends the entire length of injection portion 32 and fluidly communicates with catheter 42. The total wall thickness is approximately in the range of 2 - 4mm.

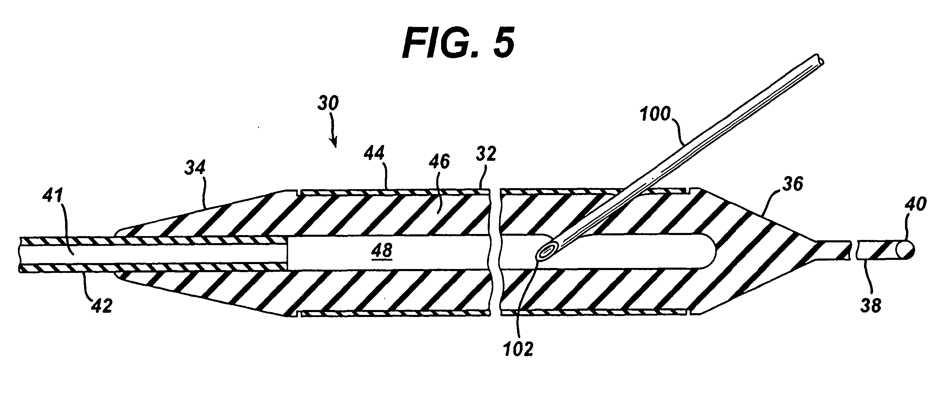

FIG. 5 is a longitudinal sectional view of flexible injection port 30, showing a hypodermic needle 100 penetrating through injection portion 32 so that distal tip 102 of hypodermic needle 100 is inside of fluid reservoir 48. First end 34, second end 36, tether 38, eye loop 40, and inner tube 46 are integrally molded from an elastomer such as, for example, silicone rubber, latex rubber, or polyurethane rubber. The molded elastomer may have a durometer approximately in the range of 40-60, but is not limited to that range. Catheter 42 may be bonded inside of first end 34 using any one of a number of bonding agents and techniques well known in the art, in order to fluidly communicate with reservoir 48. Outer tube 44 may be made of a PTFE shrink-wrap material, or a similar, biocompatible shrink-wrap. During the manufacturing process, outer tube 44 may be loosely assembled in the pre-shrunken configuration over inner tube 46. Then the application of heat causes outer tube 44 to conform very tightly around inner tube 46. Outer tube 44 therefore applies a significant compressive force on the softer, inner tube 46 to enhance the ability of inner tube 46 to close the puncture created by hypodermic needle 100.

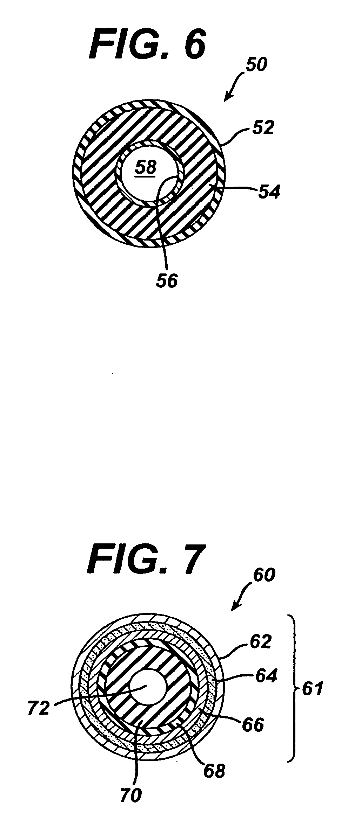

FIG. 6 is a cross sectional view of a second example of a flexible injection port 50, which is externally similar to the first example shown in FIG. 3. Flexible injection port 50 includes an outer tube 52, an inner tube 54, and an inner lining 56. Outer tube 52 and inner tube 54 are the same as outer tube 44 and inner tube 46, respectively, of the first example in FIG. 4. Inner lining 56 may be an extruded plastic, thin wall tube, such as polyethylene or PTFE, tightly assembled inside of inner tube 54 to provide internal support to inner tube 54. By supporting inner tube 54 in this way, a greater compressive force may be applied by outer tube 52 onto inner tube 54, to further enhance the self-sealing capability. The material of inner lining 56 may be selected to have a higher needle penetration resistance than inner tube 54. This difference in penetration resistance provides the surgeon with tactile feedback that the needle tip has penetrated into fluid reservoir 58. Inner lining 56 may also be constructed of a metallic mesh and be similar in many respects to a vascular stent. Again, the total wall thickness is approximately in the range of 2-4 mm.

FIG. 7 is a cross sectional view of a third example of a flexible injection port 60, which also is externally similar to the first example shown in FIG. 3. Flexible injection port 60 comprises a plurality of layers 61, which for this third example includes a first layer 62, a second layer 64, a third layer 66, a fourth layer 68, and a fifth layer 70, which surrounds a fluid reservoir 72. Once penetrated by a needle that is inserted at an acute angle, the punctures created through the layers are not aligned to allow leakage once the needle is removed. Each of layers 61 may be made of the same or a different material than any of the other of layers 61, or may have the same or a different thickness than any of the other of layers 61. Each of layers 61 may have a specific property or functional contribution. For example, first layer 62 may be made of a material that is permeable to tissue fluids in order to slowly release a medication contained in second layer 64. Fifth layer 70 may be made of silicone rubber having a durometer in the range of 20 - 30. Fourth layer 68 may be made of a heat shrinkable PTFE material, which applies a radially compressive force on fifth layer 70 to enhance self-sealing. Third layer 66 may be made of a material such as a metallic foil that acts as a diffusion barrier to prevent the loss of fluid from fluid reservoir 72. Fourth layer 66 may be made of a high durometer silicone rubber. Many other materials are possible, in a multiplicity of combinations, so that injection port 60 may have characteristics especially suited for its particular application. Diffusion of body fluids into and out of the soft port wall may also be reduced by any one of various material treatment techniques, including, for example, vapor deposition of titanium or another metal on a surface of the soft port, and coating with *Paralene* polymer. Other coatings are also known in the art for micro bacterial protection. Again, the total wall thickness is in the range of 2 - 4 mm.

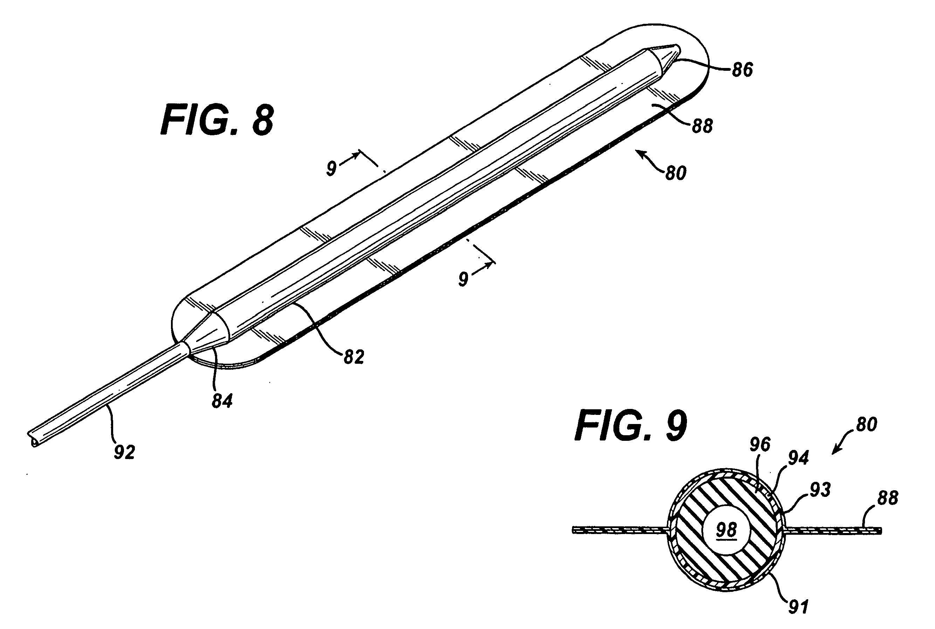

FIG. 8 is a fourth example of a flexible injection port 80, which comprises the present invention. The flexible injection port 80, comprises a first end 84 that attaches to a catheter 92, a second end 86 and an injection portion 82. Flexible injection port 80 further comprises a webbing 88 attached to and covering at least injection portion 82, and made of a thin, flexible, implantable material such as a polyester or polypropylene mesh, expanded PTFE, or the like. Webbing 88 provides broad margins for stapling or suturing to an underlying tissue such as fascia, as well as a large area for tissue in-growth, to enhance long-term stability and to substantially prevent migration of flexible injection port 80. FIG. 9 is a cross sectional view of flexible injection port 80, taken at line 9-9 of FIG. 8. Flexible injection port 80 comprises an outer tube 94 made of a heat shrinkable, PTFE material, and an inner tube 96 made of a silicone rubber having a durometer of approximately 20-40. Webbing 88 includes a pair of webbing layers, 91 and 93, bonded thermally or chemically tightly over at least injection portion 82 in the mid-plane of flexible injection port 80.

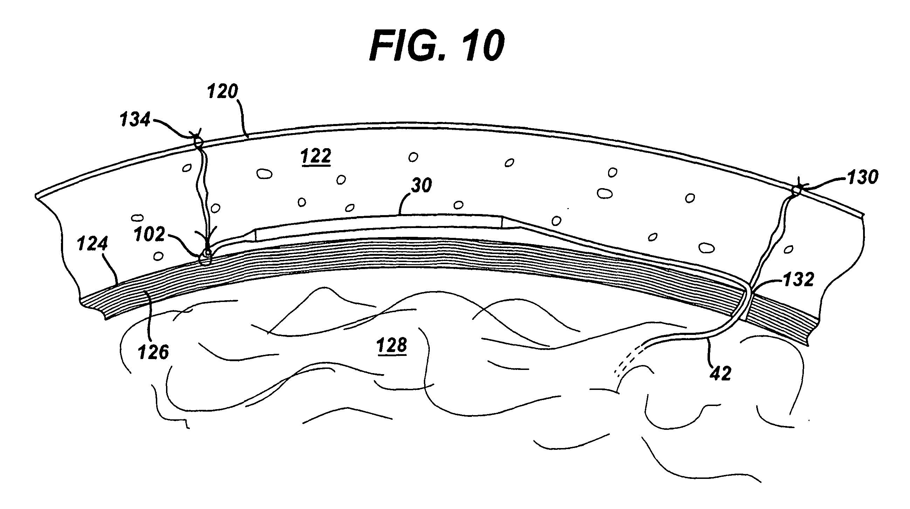

A surgeon may implant the flexible injection port, as described for the preceding examples, in a number of locations in a patient's body. FIGs 10, 11, and 12 show examples of flexible injection port 30 subcutaneously implanted in the abdomen of a patient, although it is possible to implant flexible injection port 30 beneath the skin in other portions of the body.

FIG. 10 depicts a first example of flexible injection port 30 subcutaneously implanted in a patient's body. Flexible injection port 30 lies adjacent to a fascia layer 124 covering an abdominal wall 126. Catheter 42 passes from the abdominal cavity 128 through an abdominal opening 132, which the surgeon used together with a first incision 130 for laparoscopic access earlier in the surgical procedure. The surgeon optionally may make a second incision 134 offset from first incision 130, and use conventional, surgical grasping and retracting instruments to pull flexible injection port 30 beneath a fat layer 122 and adjacent to fascia layer 124. However, the surgeon may determine that it is not necessary to make a second incision 134, and instead use first incision 130 to push flexible injection port 30 into position. In either situation, the surgeon dissects as little tissue as practical in order to save surgery time and to minimize the size of enclosed cavities that may collect tissue fluids and become sites for infection. The surgeon optionally may anchor flexible injection port 30 to fascia layer 124 with a stay suture 102. Once the surgeon has placed flexible injection port 30 in the desired location, the surgeon closes first incision 130 and second incision 134 using conventional sutures or staples.

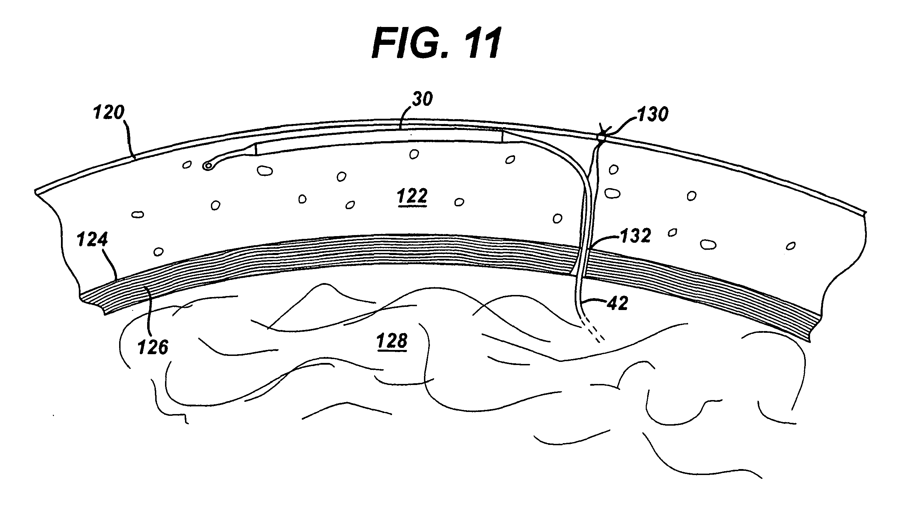

FIG. 11 shows a second example of flexible injection port 30 subcutaneously implanted in a patient's body. Flexible injection port 30 lies immediately beneath skin layer 120 and above fat layer 122. Catheter 42 passes through first incision 130 and abdominal opening 132 (the original laparoscopic port site) into abdominal cavity 128. The surgeon may use finger or instrument dissection through first incision 130 to create a space under skin layer 120 for flexible injection port 30. The surgeon closes first incision 130 using conventional sutures or staples. Normally it would not be necessary to close abdominal opening 132 through fascia layer 124 and abdominal wall 126, but the surgeon may do so in order to promote healing and to prevent slippage of catheter 42 through abdominal opening 132. The surgeon may prefer placement of flexible injection port 30 just beneath skin layer 120 for severely obese patients in which fat layer 122 is over 5-10 cm thick, so that the surgeon may easily use palpation to locate flexible injection port 30 for later injections of fluid. Also, conventional intravenous (IV) needles and techniques may be used for injecting the fluid into flexible injection port 30, which is situated beneath the skin much like a natural blood vessel. This may allow nurses and other clinicians who are trained in administering IV's to assist the surgeon with fluid injections. Furthermore, if the clinician uses a conventional IV needle, the "flashback" of fluid into the IV needle syringe tip provides the clinician with visual feedback that the tip of the needle is properly penetrated into the reservoir of flexible injection port 30. In fact, addition of a colorant to the fluid injected further enhances this visual feedback. Non-toxic colorants that may be added to the saline solution or medication are well known in the art.

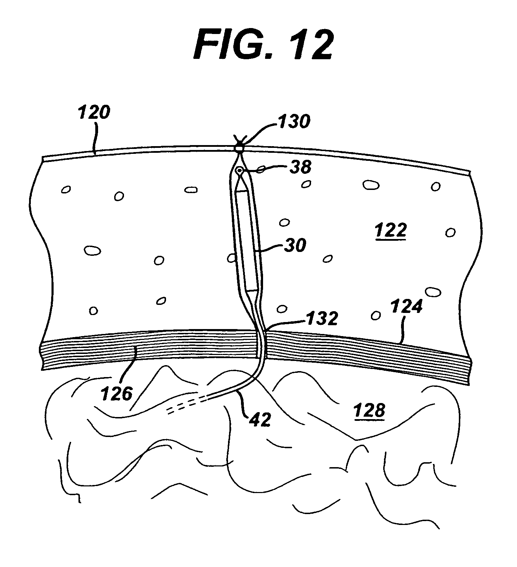

FIG. 12 shows a third example of flexible injection port 30 subcutaneously implanted in a patient's body. For this example, the surgeon does minimal or no dissection of tissue at the laparoscopic port site. Catheter 42 passes from the abdominal cavity 128 through fascia layer 124 and abdominal wall 126. The surgeon positions flexible injection port 30 vertically in fat layer 122 and beneath skin layer 120. Optionally, the surgeon may suture abdominal opening 132 to prevent slippage of flexible injection port 30 into abdominal cavity 128. The surgeon also may use a surgical scissors to trim off tether 38 from flexible injection port 30, just prior to closing first incision 130 with conventional sutures or staples.

The present invention, a flexible injection port, as described in Figs. 8/9, has numerous advantages over the prior art injection ports. The flexible injection port may not require attachment to fascia, thus reducing the duration of the surgical procedure. The flexible injection port may require a smaller incision size and less tissue dissection for implantation, so that the patient has less pain, less scarring, a faster recovery, and less possibility of infection. Due to the integral construction of the flexible injection port and the catheter, the step of connecting the catheter to the injection port during the surgical procedure is not necessary, thus potentially reducing the number of surgical complications due to fluid leakage at the connection. Because the flexible injection port may be implanted in the fat layer near the skin surface, the surgeon or a trained clinician may use palpation to locate the injection port, and standard IV techniques to administer fluid, yet the implant is still cosmetically acceptable to the patient. In addition, shorter injection needles may be used to reduce patient anxiety during fluid administration. The flexible injection port may have no metallic parts, resulting in a flexible and lightweight implant for greater patient comfort and compatibility with magnetic resonance and fluoroscopic x-ray imaging. Finally, the injection portion of the flexible injection port is accessible with a hypodermic needle for most of the possible orientations of the flexible injection port within the subcutaneous fat layer of the patient.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, the injection port may me coated with an anit-microbial coating such as triclosan. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. An injection port (80) for subcutaneous placement within a body comprising:
a. an elongated flexible substantially non-rigid body (82) having first and second ends (84,86) and a wall therebetween, said wall is such that it will self seal after being punctured, said body further including a fluid reservoir (98) surrounded by said wall; and
b. a flexible elongated tubular catheter (92) attached to said body (82) which is in fluid communication with said reservoir,
wherein the flexible injection port (80) further comprises a webbing (88) attached to and extending from an outer surface of said body (82), **characterised in that**:
the webbing (88) comprises a pair of webbing layers (91, 93) bonded thermally or chemically tightly over the body (82) in a mid-plane of the flexible injection port (80).

2. The flexible injection port of Claim 1 wherein said wall comprises an inner layer (96) and an outer layer (94), wherein said outer layer is in compression around said inner layer.

3. The flexible injection port of Claim 2, wherein each of said inner layer (96) and said outer layer (94) are made from a polymer material.

4. The flexible injection port of Claim 2 wherein said outer layer (94) is made of a heat shrinkable material, and said inner layer (96) is made of an elastomer.

5. The flexible injection port of Claim 1, wherein said body (82) has a diameter no greater than 12mm.

6. The flexible injection port of any preceding claim, wherein the substantially non-rigid body (82) is tubular.

7. The flexible injection port of claim 9, wherein said body (82) has a length between said ends of about 5mm to about 20mm and an outer diameter from about 5mm to about 12mm.

## Patentansprüche

1. Injektionsöffnung (80) zur subkutanen Platzierung in einem Körper, die Folgendes umfasst:
a. einen länglichen flexiblen, im Wesentlichen nicht starren Körper (82) mit ersten und zweiten Enden (84, 86) und einer Wand dazwischen, wobei die Wand so beschaffen ist, dass sie sich nach Durchstechen von selbst versiegelt, wobei der Körper ferner ein von der Wand umgebenes Flüssigkeitsreservoir (98) umfasst; und
b. einen flexiblen länglichen röhrenförmigen Katheter (92), der am Körper (82) befestigt ist und mit dem Reservoir in Flüssigkeitsverbindung steht,
worin die flexible Injektionsöffnung (80) ferner ein Gewebe (88) umfasst, das an einer Außenseite des Körpers (82) befestigt ist und sich von dieser aus erstreckt; **dadurch gekennzeichnet, dass** das Gewebe (88) ein Paar Gewebeschichten (91, 93) umfasst, die thermisch oder chemisch fest über dem Körper (82) in einer Mittelebene der flexiblen Injektionsöffnung (80) aufgeklebt sind.

2. Flexible Injektionsöffnung nach Anspruch 1, worin die Wand eine Innenschicht (96) und eine Außenschicht (94) umfasst, worin die Außenschicht um die Innenschicht komprimiert ist.

3. Flexible Injektionsöffnung nach Anspruch 2, worin jede Innenschicht (96) und die Außenschicht (94) aus einem Polymermaterial bestehen.

4. Flexible Injektionsöffnung nach Anspruch 2, worin die Außenschicht (94) aus einem Schrumpfmaterial besteht und die Innenschicht (96) aus einem Elastomer besteht.

5. Flexible Injektionsöffnung nach Anspruch 1, worin der Körper (82) einen Durchmesser von höchstens 12 mm aufweist.

6. Flexible Injektionsöffnung nach einem der vorhergehenden Ansprüche, worin der im Wesentlichen nicht starre Körper (82) röhrenförmig ist.

7. Flexible Injektionsöffnung nach Anspruch 9, worin der Körper (82) eine Länge zwischen den Enden von ca. 5 mm bis ca. 20 mm und einen Außendurchmesser von ca. 5 mm bis ca. 12 mm aufweist.

## Revendications

1. Orifice d'injection (80) destiné à être positionné sous la peau dans un corps qui comporte :
a. un corps (82) allongé flexible sensiblement non rigide présentant une première et une deuxième extrémités (84, 86) et une paroi entre les deux, ladite paroi est telle qu'elle se referme toute seule après avoir été perforée, ledit corps comprenant en outre un réservoir pour fluide (98) entouré par ladite paroi ; et
b. un cathéter (92) tubulaire allongé flexible fixé audit corps (82) qui est en communication fluidique avec ledit réservoir,
dans lequel ledit corps d'injection (80) flexible comporte en outre une toile (88) fixée à une surface externe dudit corps (82) et s'étendant depuis ladite surface externe, **caractérisé en ce que** :
la toile (88) comporte deux couches de toile (91, 93) liées thermiquement ou chimiquement de manière étroite sur le corps (82) dans une partie médiane de l'orifice (80) d'injection flexible.

2. Orifice d'injection flexible selon la revendication 1 dans lequel ladite paroi comporte une couche interne (96) et une couche externe (94), ladite couche externe étant en compression autour de ladite couche interne.

3. Orifice d'injection flexible selon la revendication 2, dans lequel chacune desdites couche interne (96) et couche externe (94) est réalisée dans un matériau polymère.

4. Orifice d'injection flexible selon la revendication 2 dans lequel ladite couche externe (94) est réalisée dans un matériau thermorétrécissable, et ladite couche interne (96) est réalisée dans un élastomère.

5. Orifice d'injection flexible selon la revendication 1, dans lequel ledit corps (82) a un diamètre qui n'est pas supérieur à 12 mm.

6. Orifice d'injection flexible selon l'une quelconque des revendications précédentes, dans lequel le corps (82) sensiblement non rigide est tubulaire.

7. Orifice d'injection flexible selon la revendication 9, dans lequel ledit corps (82) a une longueur entre lesdites extrémités d'environ 5 mm à environ 20 mm et un diamètre extérieur d'environ 5 mm à environ 12 mm.
